# EUROPEAN PATENT SPECIFICATION

(11) **EP 1 919 477 B1**
(45) Date of publication and mention of the grant of the patent: **11.07.2012**
(21) Application number: 06774700.6
(22) Date of filing: 26.07.2006
(51) Int. Cl.: A61K 31/44, A61P 43/00

(54) **Use of [2-(2-methylimidazol-1-yl)methyl]pyridine for treating sleep disorders**
Verwendung von [2-(2-methylimidazol-1-yl)methyl]pyridin zur Behandlung von Schlafstörungen
Utilisation du [2-(2-methylimidazol-1-yl)methyl]pyridine pour le traitement des troubles du sommeil

(30) Priority: 29.07.2005 US 703604 P; 02.11.2005 US 732536 P
(43) Date of publication of application: 14.05.2008
(73) Proprietor: Vanda Pharmaceuticals Inc., Rockville, MD 20850 (US)
(72) Inventor: LAVEDAN, Christian, N., Potomac, MD 20854 (US); POLYMEROPOULOS, Mihael, H., Potomac, MD 20854 (US)
(74) Representative: Hofstetter, Alfons J.
(86) International application number: PCT/US2006/029112
(87) International publication number: WO 2007/016203

(56) References cited:
- EP-A- 0 534 904
- WO-A-01/78726
- US-A- 5 635 521
- US-A- 5 856 343
- US-A- 5 856 343
- VACHHARAJANI N.N. ET AL.: 'Preclinical pharmacokinetics and metabolism of BMS-214778, a novel melatonin receptor agonist' JOURNAL OF PHARMACEUTICAL SCIENCES vol. 92, no. 4, April 2003, pages 760 - 772, XP008074135

## Description

### BACKGROUND OF THE INVENTION

### (1) Technical Field

The present invention relates generally to the treatment of excessive sleepiness. The present invention also relates generally to the treatment of diseases wherein excessive sleepiness is a contributing factor or a complicating condition associated with another disease or following anesthesia.

### (2) Description of Related Art

Various compounds are known to or suspected of improving wakefulness in individuals. For example, U.S. Patent Application Publication No. 20040143021 describes the use of modafinil to improve wakefulness following the administration of general anesthesia. U.S. Patent Application Publication No. 20010034373 similarly describes the administration of modafinil to improve cognitive function.

US 5 856 343 A and EP 0 534 904 A1 disclose the use of imidazolylmethylpyridines for the treatment of senile dementia, Alzheimer's disease and depression.

WO 01/78726 A1 discloses the use of imidazolylmethylpyridines as cytoprotective and cell rescuing agent or for the treatment of obesity.

### SUMMARY OF THE INVENTION

The invention relates to the use in mammalian patients and especially in human patients, of certain imidazolylalkyl-pyridines as wakefulness compounds to treat or prevent excessive sleepiness associated with narcolepsy, obstructive sleep apnea/hypopnea syndrome, shift work sleep disorder, desynchronization disorders, ovulation disorders, seasonal melancholia, jet lag (time zone syndrome) or wakefulness disturbances as a consequence of jet lag, and to assist, alone or in conjunction with certain sleep inducing agents, in modulating circadian rhythmicity dysfunctions due to shift work, aging, blindness, jet-lag, exposure to sub-arctic days and nights, or other environmental circumstances.

More particularly, the invention relates to such uses of [2-(2-methylimidazol-1-yl)methyl]pyridine in free base or acid addition salt form. Also, compounds of formula I, which do not form a part of the invention, are disclosed 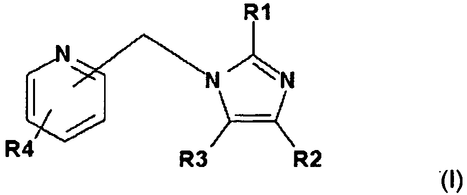 wherein R₁ is hydrogen, lower alkyl, halogen with an atomic number of 9 to 35 or amino optionally mono- or disubstituted by lower alkyl, R₂ and R₃ independently of one another are hydrogen or lower alkyl, R₄ is hydrogen, hydroxy, lower alkyl, lower alkoxy or halogen with an atomic number of 9 to 35, in free base or acid addition salt form, and the bridge between the pyridine and the imidazole, illustrated as methylene, is methylene or ethylene.

Also, a method of altering wakefulness and sleep is disclosed that comprises administering to the patient an effective amount of an imidazolylmethyl-pyridine. The imidazolylmethyl-pyridine may be administered alone or co-administered with an effective amount of a sleep-inducing agent, or, alternatively, co-administered with an effective amount of an additional wakefulness promoting agent. Exemplary sleep-inducing agents include melatonin agonists.

Further, a method is disclosed of treating or preventing excessive sleepiness associated with narcolepsy, obstructive sleep apnea/hypopnea syndrome, jet lag or wakefulness disturbances as a consequence of jet lag, or diseases of the nervous system, that comprises administering to the patient an effective amount of [2-(2-methylimidazol-1-yl)methyl]pyridine.

The foregoing and other features of the invention will be apparent from the following more particular description of embodiments of the invention.

### DETAILED DESCRIPTION OF THE INVENTION

As used herein, "lower," in the context of alkyl and alkoxy groups, denotes a radical having up to 7 carbon atoms, preferably up to 4 carbon atoms and more preferably up to 2 carbon atoms. Consequently, lower alkyl has especially up to 7 carbon atoms, preferably up to 4 carbon atoms, and in particular up to 2 carbon atoms and is, for example, methyl, ethyl, propyl, isopropyl, butyl, tert-butyl, pentyl, or hexyl. Accordingly, lower alkoxy has up to 7 carbon atoms, preferably up to 4 carbon atoms, and in particular up to 2 carbon atoms and is, for example, methoxy, ethoxy, propoxy, butoxy, tert-butoxy or hexyloxy.

Insofar as above-defined, lower alkyl or lower alkoxy groups present in the compounds of formula (I), preferably have one or two carbon atoms and especially signify methyl or methoxy. The imidazolylmethyl radical is preferably in position 2 of the pyridine. R₁ is preferably methyl or ethyl, more preferably methyl. R₂ and R₃ are preferably each hydrogen. R₄ is preferably methyl, ethyl or hydrogen, more preferably methyl or hydrogen, and in particular hydrogen. The compound A of Example 1, namely [2-(2-methylimidazol-1-yl)methyl]pyridine, is the pharmaceutically active compound according to the invention.

In a particular group of compounds of formula (I), R₁ is lower alkyl, R₂ and R₃ independently of one another are hydrogen or lower alkyl, and R₄ is hydrogen, lower alkyl or halogen with an atomic number of 9 to 35.

In a further particular group of compounds of formula (I), R₁ is methyl, R₂ and R₃ independently of one another are hydrogen or methyl, and R₄ is hydrogen, methyl or halogen with an atomic number of 9 to 35.

Halogen with an atomic number of 9 to 35 denote in particular a fluorine and chlorine residue, more particularly a chlorine residue.

The compounds of formula (I) may be present in free base form or in the form of their acid addition salts, including, for example, hydrogen fumarate and fumarate salt forms. Acid addition salts may be produced from the free bases in known manner, and vice versa.

The compounds of formula (I) are known, e.g., from U.S. Patent Nos. 5,856,343 and 5,635,521, or may be produced in accordance with known processes, i.e., analogously to known processes.

U.S. Patent No. 5,856,343 describes synthesis of Compound A, [2-(2-methylimidazol-1-yl)methyl]pyridine, as follows:

9.7 g (75 mM) of 2-(chloromethyl)pyridine and 42 g (512 mM) of 2- methylimidazole are suspended in 40 ml dimethylformamide, then stirred for 3 hours at 105 °C. The dimethylformamide is distilled off and the crystalline residue is diluted with ethyl acetate and a little hexane. Following filtration, the mother solution is concentrated by evaporation and the dimethylformamide distilled off, and then shaken out several times between water and methylene chloride. 10.3 g of the oily title compound are obtained.

[2-(2-methylimidazol-1-yl)methyl]pyridine and its physiologically acceptable salts exhibit interesting effects on the wakefulness of a subject and may therefore be used in accordance thereto. The compounds according to the invention are therefore useful to treat excessive sleepiness associated with narcolepsy, obstructive sleep apnea/hypopnea syndrome, or diseases of the nervous system and to assist, in conjunction with certain sleep inducing agents, in modulating circadian rhythmicity dysfunctions due to shift work, aging, blindness, jet-lag, exposure to sub-arctic days and nights, or other environmental circumstances.

As used herein, "Melatonin agonist or melatonin agonists", refer to bioactive molecules that bind one-or more melatonin receptors, preferably MT 1 and MT2, in mammals and exhibit effects on sleep and circadian rhythm following administration. They include acidic, basic, zwitterions, or neutral bioactive molecules and their salts and derivatives that bind one or more melatonin receptors in mammals and exhibit on sleep and circadian rhythm following administration. Derivatives include prodrugs or metabolites of a melatonin agonist, or salts, solvates, hydrates, bioactive stereoisomers, or crystalline or amorphous forms of melatonin agonists; for example MA-1 (see Vachharajani et al., J. Pharmaceutical Sci., 92(4):760-772 (19), and hydroxylated, dehydrogenated, glucuronide and diol derivatives. Exemplary members of the melatonin agonist class of compounds is Melatonin Agonist-1, (1 R-Trans)-N-[[2-(2,3-dihydro-4-benzofuranyl)cyclopropyl]methyl] propan- amide, Melatonin Agonist-2 (N-[1-(2,3-dihydrobenzofuran-4-yl)pyrrolidin-3-yl]-N-ethylurea]), and ramelteon, (S)-N-[2-(1,6,7,8-tetrahydro-2H-indeno[5,4-b]furan-8-yl) ethyl] propionamide, the composition and structure of which is disclosed in PCT Pat. Publ. WO 97/32871. Additional exemplary members of the class are LY156735, (R)-N-(2-(6-chloro-5-methoxy-1H-indol-3yl)propyl)acetamide, GR196429, N-[2,3,7,8-tetrahydro-1H-furo(2,3-g)indol-1-yl]ethyl] acetamide (see Beresford et al., J. Pharmacol. and Exp. Therapeutics, 285: 1239-45 (1998) for structure and characterization), S-20098 (agomelatine; see Yous et al., J. Med. Chem., 35: 21484-86 (1992) for structure, synthesis and characterization) and 2-Bromomelatonin, (N-[2-(2-bromo-5-methoxy-1H-indol-3-yl)ethyl]-acetamide (see Duranti, et al., Life Sci., 51:479-85 (1992) for synthesis and characterization). Still others include those melatonin agonists described in Spadoni et al., J. Med. Chem., 36: 4069-74 (1993) (5-methoxy-N-acyltryptamines), in Langlois et al., J. Med. Chem., 38: 2050-60 (1995) (N-[2-(2-methoxy-naphthyl)ethyl]propionamide, H-[2-(2,7-dimethoxy naphthayl) ethyl]cyclopropylformamide) and in Copinga et al., J. Med. Chem., 36: 2891-98 (1993) (2-acetamido-8-methoxytetralin).

As used herein the terms "co-administration", "co-administered" and "co-administer" refer to the administration of one of the wakefulness-inducing agents of the invention in conjunction with one or more additional active pharmaceutical ingredients in a manner that results in the desired therapeutic effect. For example, administration of a sleep-inducing agent and administration of a wakefulness-inducing agent within a 24 hour period is considered herein "co-administration".

As used herein the terms "treatment" or "treat" refer to both prophylactic or preventive treatment as well as curative or disease-modifying treatment, including treatment of patients at risk of contracting the disease or suspected to have contracted the disease as well as patients who are ill or have been diagnosed as suffering from a disease or medical condition.

The compounds according to the invention may be administered by any conventional route, in particular enterally, orally, or topically, for example in the form of tablets, capsules or eye drops, or parenterally, for example in the form of injectable solutions or suspensions.

An effective amount of the active agent of the invention, namely [2-(2-methylimidazol-1-yl)methyl]pyridine, may be administered to a subject animal (typically a human but other animals, *e.g.*, farm animals, pets and racing animals, can also be treated). An effective amount is an amount that prevents, inhibits, or terminates excessive sleepiness (ES) associated with narcolepsy, obstructive sleep apnea/hypopnea syndrome (OSAHS), jet lag or wakefulness disturbances as a consequence of jet lag, or other disorders, hypersomnia, REM behavior disorder, insomnia, parasomnia, sleep-related diagnostic dilemma, sleep apnea associated with neurological disorders, shift worker sleep disorder (SWSD), Kleine-Levin syndrome, and sleep/wake disorders in blind subjects. An effective amount is an amount that also prevents, inhibits, or terminates sleep disturbances induced by neurological injuries, abnormalities, lesions, or surgery.

Sleep disorders, and particularly REM sleep disturbances, are also known to be associated with various psychiatric disorders, including mania, seasonal affective disorder, bipolar disorder, and schizophrenia. [2-(2-methylimidazol-1-yl)methyl]pyridine has been shown to affect the wake-sleep cycle in general and is particularly effective in decreasing REM sleep. Thus, in particular embodiments, a method of treating or preventing a sleep disorder associated with depressive disorders, mania, seasonal affective disorder, bipolar disorder, or schizophrenia is disclosed.

For the indications as described herein, the appropriate dosage will, of course, vary depending upon, for example, the compound employed, the host, the mode of administration and the nature and severity of the condition being treated. However, in general, satisfactory results in animals are indicated to be obtained at daily dosages from about 0.05 to about 50 mg/kg animal body weight. In larger mammals, for example humans, an indicated daily dosage may typically range from about 0.1 mg to about 1600 mg, more typically about 1 mg to about 800 mg, or about 10 mg to about 200 mg, conveniently administered, for example, in divided doses up to four times a day. The active agent could be administered either before the sleep disturbance (to prevent or minimize its effect) or after (to terminate or diminish its effect, and to improve and accelerate the recovery).

The wakefulness promoting agents of the invention may be co-administered in effective dosages with effective dosages of sleep-inducing agents in order to modulate the amount and/or timing of wake and sleep, for example in the case of circadian rhythmicity dysfunctions due to shift work, aging, blindness, jet-lag, exposure to sub-arctic days and nights, or other environmental circumstances. In this context, the sleep-inducing agent would be administered to promote sleep at an appropriate time and the wakefulness promoting agent would be administered to promote wakefulness at the appropriate time, thereby modifying the patient's sleep-wake cycle. For such uses, the sleep-inducing agent and the wakefulness-promoting agent can be packaged together, e.g., in "day-night" packaging so that it is convenient for the patient to know which drug to use at what time of the day. Examples of other sleep-inducing agents include among others melatonin agonists, eszopiclone, zolpidem, zopiclone, brotizolam and triazolam.

The wakefulness promoting agents of the invention may also be co-administered in effective dosages with effective dosages of other wakefulness-promoting agents to enhance the wakefulness promoting or other effects. For example, the wakefulness promoting agents of the invention can be co-administered with modafinil or armodafinil or stimulants to enhance or complement the effects of both agents.

The appropriate effective amount of a sleep-inducing agents when co-administered with the wakefulness promoting agents of the invention in order to modulate the amount and/or timing of wake and sleep is that amount which results in the desired effect of inducing sleep in the patient and, of course, will vary depending upon, for example, the compound employed, the host, the mode of administration and the nature and severity of the condition being treated. In animals satisfactory results may be obtained at daily dosages from about 1 to about 500 mg/kg animal body weight. In larger mammals, for example humans, an indicated daily dosage may typically range from about 1 mg to about 300 mg, more typically about 10 mg to about 200 mg, or about 10 mg to about 150 mg. For best results, the sleep-inducing agent should be administered within about an hour of bedtime and the wakefulness-inducing agent administered within about an hour of wakefulness or about 8 to about 10 hours post-sleep inducing agent administration.

It will be understood that the amount of the compound actually administered will be determined by a physician, in the light of the relevant circumstances including the condition to be treated, the choice of compound to be administered, the chosen route of administration, the age, weight, and response of the individual patient, and the severity of the patient's symptoms.

For therapeutic or prophylactic use, an imidazolylmethyl-pyridine will normally be administered as a pharmaceutical composition comprising as the (or an) essential active ingredient at least one such compound in association with a solid or liquid pharmaceutically acceptable carrier and, optionally, with pharmaceutically acceptable adjuvants and excipients employing standard and conventional techniques.

The pharmaceutical compositions include suitable dosage forms for oral, parenteral (including subcutaneous, intramuscular, intradermal, intravenous, transdermal (such as via a dermal patch, gel, microneedle, iontophoresis, sonophoresis, or phonophoresis), bronchial or nasal administration. Thus, if a solid carrier is used, the preparation may be tableted, placed in a hard gelatin capsule in powder or pellet form, or in the form of a troche or lozenge. The solid carrier may contain conventional excipients such as binding agents, fillers, tableting lubricants, disintegrants, wetting agents and the like. The tablet may, if desired, be film coated by conventional techniques. If a liquid carrier is employed, the preparation may be in the form of a syrup, emulsion, soft gelatin capsule, sterile vehicle for injection, an aqueous or non-aqueous liquid suspension, or may be a dry product for reconstitution with water or other suitable vehicle before use. Liquid preparations may contain conventional additives such as suspending agents, emulsifying agents, wetting agents, non-aqueous vehicle (including edible oils), preservatives, as well as flavoring and/or coloring agents. For parenteral administration, a vehicle normally will comprise sterile water, at least in large part, although saline solutions, glucose solutions and like may be utilized. Injectable suspensions also may be used, in which case conventional suspending agents may be employed. Conventional preservatives, buffering agents and the like also may be added to the parenteral dosage forms. Particularly useful is the administration of [2-(2-methylimidazol-1-yl)methyl]pyridine in oral dosage formulations. The pharmaceutical compositions are prepared by conventional techniques appropriate to the desired preparation containing appropriate amounts of the active ingredient, that is, [2-(2-methylimidazol-1-yl)methyl]pyridine according to the invention. See, for example, Remington's Pharmaceutical Sciences, Mack Publishing Company, Easton, Pa., 17th edition, 1985.

In making pharmaceutical compositions containing compounds of the present invention, the active ingredient(s) will usually be mixed with a carrier, or diluted by a carrier, or enclosed within a carrier, which may be in the form of a capsule, sachet, pamper, or other container. When the carrier serves as a diluent, it may be a solid, semisolid, or liquid material which acts as a vehicle, excipient or medium for the active ingredient. Thus, the composition can be in the form of tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin capsules, suppositories, sterile injectable solutions and sterile packaged powders.

Some examples of suitable carriers and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, methyl cellulose, methyl- and propylhydroxybenzoates, talc, magnesium stearate and mineral oil. The formulations can additionally include lubricating agents, wetting agents, emulsifying and suspending agents, preserving agents, sweetening agents, or flavoring agents. The compositions of the invention may be formulated so as to provide quick, sustained or delayed release of the active ingredient after administration to the patient.

The compositions are preferably formulated in a unit dosage form, each dosage containing from about 0.1 to 800 mg of the active ingredient. The term "unit dosage form" refers to physically discrete units suitable as unitary dosages for human subjects and other mammals, each unit containing a predetermined quantity of active material calculated to produce the desired therapeutic effect, in association with the required pharmaceutical carrier. For co-administration, a unit dosage form of a sleep-inducing agent would contain from about 10 to about 200 mg of the active ingredient.

Several experiments, described below, demonstrate the ability of various salt forms and various doses of an imidazolylmethyl-pyridine such as Compound A ([2-(2-methylimidazol-1-yl)methyl]pyridine) at altering sleep cycle and wakefulness.

### 1. Materials and Methods

### 1.1 Compound

In a first set of experiments, the oral (p.o.) and subcutaneous (s.c.) actions of Compound A were evaluated in the sleep-wakefulness cycle in rats. For this purpose the salt forms multifumarate (mfu) or fumarate (fu) were used.

In a second set of experiment, oral actions of Compound A were evaluated in the sleep-wakefulness cycle in rats with lesions of the nucleus basalis of Meynert (NBM) and locus coeruleus (LC). For this purpose the salt form multifumarate (mfu) was used.

### 1.2 Animal studies

### 1.2.1 Non-lesioned rats

The experiments were carried out in nonanesthetized unrestrained male Wistar rats prepared with chronically implanted electrodes for the recording of the electrocorticogram (ECoG). The animals were caged in a room, maintained at a constant temperature of 22 °C and artificially lighted for 12 hours daily. Before beginning of the experiment, the animals were given a 1 to 3 day period of acclimatization in the recording cages and had complete freedom of movement even when the recording cables were attached. The ECoG recordings were made continuously during 22 to 23-hours period, 15 min after drug administration. Each animal served as its own control. In one experiment, recordings of EcoG were also obtained after administration of distilled water (vehicle used with Compound A) for comparison with no administration, or administration of Compound A.

### 1.2.2 Lesioned rats

Male rats were used. They were anesthetized with pentobarbital (50 mg/kg i.p.) and positioned in a stereotaxic apparatus with the upper incisor bar set 5 mm (locus coeruleus, LC) or 3.3 mm (Nucleus basalis of Meynert, NBM) below the interaural line. The lesions were carried out with a radio frequency lesion generator at 60 °C in 10 sec. A corresponding group of sham lesioned rats was carried out. The animals were individually kept in single cage during 6 months after the lesions. Five months after lesion, cortical electrodes were implanted. The animals were caged in a room, maintained at a constant temperature of 22 °C and artificially lighted for 12 hours daily. Before beginning of the experiment, the animals were given a 1 day period of acclimatization in the recording cages. These were tall plastic cylinders within the animals had complete freedom of movement even when the recording cables were attached. ECoG recordings were made continuously during 22-hours period/day, 15 min after the installation of the animal or 15 min after drug administration.

Four groups of animals were studied (A1-4), and statistical analysis (T-test, p<0.05) was performed between the different groups.
A1: 5 lesioned animals, which had received 35 days after lesion 3 mg/kg p.o. of Compound A during 5 days. Implementation of electrodes 5 months later. Recording of ECoG on the 22d and 23d days after implantation.
A2: Same as A1 but treated with vehicle instead of Compound A.
A3: S Same as A1 but with Sham rats instead of lesioned animals.
A4: Same as A3 but treated with vehicle instead of Compound A.

### 2 Results

### 2.1 Non-lesioned rats

3, 10 and 30 mg/kg (mfu) p.o., 3, and 10 mg/kg (fu) p.o., and 1, 3, and 10 mg/kg (mfu) s.c. did induce significant changes in the rat nycthemeral rhythm in a dose dependent manner.

At 3 mg/kg p.o., Compound A reduced REM sleep duration during the first 4 hours. The classical sleep was also reduced. An increase in wakefulness was observed during this period. Compound A significantly delayed slow-wave sleep (SWS) sleep onset. Active waking tended to be increased between 0-4 and 7-11 hours and was significantly increased during the last recording interval, 18.75-23 hours.

At 10 mg/kg p.o., Compound A tended to increase waking time during the first 6 hours, decreased classical sleep during 3 hours, and significantly reduced REM sleep duration for several hours. Between 7-11 hours after administration, Compound A still significantly increased waking time and decreased SWS duration. For the same time interval, REM sleep also tended to be increased. Active waking was significantly increased between 0-4 hours after administration. Furthermore, Compound A strongly delayed REM sleep onset, and tended to also delay SWS onset.At 30 mg/kg p.o., Compound A strongly delayed both SWS and REM sleep onset. Furthermore, it increased waking time and decreased SWS and REM sleep duration during the first 4 hours of recording and in addition REM sleep duration between 4-7 hours after administration. All of the effects were very strong; the decrease of REM sleep lasted for about 11 hours, the increase of wakefulness about 9 hours.

At 1 mg/kg s.c., Compound A reduced REM sleep duration during the first 2 hours, and enhanced wakefulness during the first 4 hours.

At 3 and 10 mg/kg s.c., Compound A induced nearly the same effects as by oral administration.

The doses of fu salt form 3 and 10 mg/kg tested p.o have the same effects on the sleep wakefulness cycle in rats than that of the mfu form.

### 2.2 Lesioned rats

5 months after lesions, the sleep-wakefulness cycle of rats was very disturbed in comparison to sham rats. Lesioned animals were more awake and have less classical sleep.

In lesioned rats, treatment of Compound A induced an important reduction in the alterations due to the lesions; both effects induced by the lesions, increase of wakefulness and decrease of classical sleep are partly prevented by Compound A, and this during all the recording periods.

### 3. Discussion

The results of the experiment with non-lesioned animals demonstrate that Compound A delayed REM sleep and SWS, induced a reduction of REM sleep, a reduction of classical sleep, and an increase of wakefulness. The effect availability in this test is good, since the action induced by Compound A is quite similar by s.c. and p.o. administration.

The results of the experiment with lesioned animals demonstrate that Compound A can reduce nycthemeral cycle abnormalities induced by neuronal lesions such as the simultaneous LC + NBM lesions, and indicate that Compound A can exhibit a corrective effect several months after its last administration.

## Claims

1. Use of [2-(2-methylimidazol-1-yl)methyl]pyridine in free base or physiologically acceptable acid addition salt form, in the preparation of a pharmaceutical composition for treating a sleep disorder in a mammal.

2. The use of claim 1, wherein [2-(2-methylimidazol-1-yl)methyl]pyridine is in free base, hydrogen fumarate, or fumarate salt form.

3. The use of claim 1, wherein the mammal is human.

4. The use of claim 3, wherein the sleep disorder is at least one of: excessive sleepiness, excessive sleepiness (ES) associated with narcolepsy, obstructive sleep apnea/hypopnea syndrome (OSAHS), jet lag or wakefulness disturbances as a consequence of jet lag, hypersomnia, REM behavior disorder, insomnia, parasomnia, sleep-related diagnostic dilemma, sleep apnea associated with neurological disorders, shift worker sleep disorder (SWSD), Kleine-Levin syndrome, sleep/wake disorders in blind subjects, and a sleep disorder induced by neurological injuries, abnormalities, lesions, or surgery.

5. The use of claim 3, wherein the sleep disorder is associated with at least one disease of the nervous system selected from the group consisting of: multiple sclerosis and post-traumatic stress disorder.

6. The use of claim 3, wherein the sleep disorder is associated with at least one psychiatric disorder selected from the group consisting of: mania, seasonal affective disorder, bipolar disorder, and schizophrenia.

7. Use of [2-(2-methylimidazol-1-yl)methyl]pyridine in free base or physiologically acceptable acid addition salt form, in the preparation of a pharmaceutical composition for modulating the amount and/or timing of wake and sleep in a mammal.

## Patentansprüche

1. Verwendung von [2-(2-Methylimidazol-1-yl)methyl]pyridin in freier Basen-oder physiologisch verträglicher Säureadditionssalzform bei der Herstellung einer pharmazeutischen Zusammensetzung für die Behandlung einer Schlafstörung bei einem Säuger.

2. Verwendung nach Anspruch 1, wobei [2-(2-Methylimidazol-1-yl)methyl]pyridin in freier Basen-, Hydrogenfumarat- oder Fumaratsalzform vorliegt.

3. Verwendung nach Anspruch 1, wobei der Säuger ein Mensch ist.

4. Verwendung nach Anspruch 3, wobei die Schlafstörung mindestens eine von folgenden ist: übermäßige Schläfrigkeit, übermäßige Schläfrigkeit (ES) in Zusammenhang mit Narkolepsie, obstruktives Schlafapnoe/Hypopnoe-Syndrom (OEAHS), Jetlag oder Wachheitsstörungen als Folge von Jetlag, Hypersomnie, REM-Verhaltensstörung, Schlaflosigkeit, Parasomnie, schlafbezogenes diagnostisches Dilemma, Schlafapnoe in Zusammenhang mit neurologischen Störungen, Schichtarbeiterschlafstörung (SWSD), Kleine-Levin-Syndrom, Schlaf/Wach-Störungen bei blinden Personen und eine Schlafstörung, die durch neurologische Verletzungen, Anomalien, Läsionen oder Operation induziert ist.

5. Verwendung nach Anspruch 3, wobei die Schlafstörung mit mindestens einer Erkrankung des Nervensystems verbunden ist, die aus der Gruppe ausgewählt ist, die aus Folgendem besteht: Multipler Sklerose und posttraumatischer Belastungsstörung.

6. Verwendung nach Anspruch 3, wobei die Schlafstörung mit mindestens einer psychiatrischen Störung verbunden ist, die aus der Gruppe ausgewählt ist, die aus Folgendem besteht: Manie, saisonal affektiver Störung, bipolarer Störung und Schizophrenie.

7. Verwendung von [2-(2-Methylimidazol-1-yl)methyl]pyridin in freier Basen-oder physiologisch verträglicher Säureadditionssalzform bei der Herstellung einer pharmazeutischen Zusammensetzung zum Modulieren der Menge und/oder des Zeitpunkts von Wachheit und Schlaf bei einem Säuger.

## Revendications

1. Utilisation de [2-(2-méthylimidazole-1-yl)méthyle]pyridine, sous sa forme de base libre ou sous sa forme de sel d'addition d'acide, physiologiquement acceptable, dans la préparation d'une composition pharmaceutique, destinée à traiter un trouble du sommeil chez un mammifère.

2. Utilisation suivant la revendication 1, dans laquelle la [2-(2-méthylimidazole-1-yl)méthyle]pyridine est sous sa forme de base libre, de fumarate d'hydrogène ou de sel de fumarate.

3. Utilisation suivant la revendication 1, dans laquelle le mammifère est un être humain.

4. Utilisation suivant la revendication 3, dans laquelle le trouble du sommeil est au moins un trouble parmi : la somnolence excessive, la somnolence excessive (SE), associée à une narcolepsie, le syndrome de l'apnée / l'hypopnée obstructive du sommeil (SAHOS), les perturbations du décalage horaire ou des troubles du rythme de veille, à titre de conséquence du décalage horaire, l'hypersomnie, le trouble du comportement en sommeil paradoxal (TCSP), l'insomnie, la parasomnie, le dilemme diagnostique, lié au sommeil, l'apnée du sommeil, associée à des troubles neurologiques, le trouble du sommeil du travailleur par équipes (TSTE), le syndrome de Kleine-Levin, les troubles du rythme sommeil /veille chez les personnes aveugles et un trouble du sommeil, induit par des atteintes neurologiques, des anomalies, des lésions ou la chirurgie.

5. Utilisation suivant la revendication 3, dans laquelle le trouble du sommeil est associé à au moins une maladie du système nerveux, sélectionnée dans le groupe, se composant de : la sclérose en plaques et l'état de stress post-traumatique.

6. Utilisation suivant la revendication 3, dans laquelle le trouble du sommeil est associé à au moins un trouble psychiatrique, sélectionné dans le groupe, se composant de : la manie, le trouble affectif saisonnier, le trouble bipolaire et la schizophrénie.

7. Utilisation de [2-(2-méthylimidazole-1-yl)méthyle]pyridine, sous sa forme de base libre ou sous sa forme de sel d'addition d'acide, physiologiquement acceptable, dans la préparation d'une composition pharmaceutique, destinée à moduler la quantité et / ou le rythme de la veille et du sommeil chez un mammifère
